# EUROPEAN PATENT SPECIFICATION

(11) **EP 2 711 174 B1**
(45) Date of publication and mention of the grant of the patent: **24.10.2018**
(21) Application number: 13184442.5
(22) Date of filing: 13.09.2013
(51) Int. Cl.: B32B 7/12, B32B 15/082, B32B 15/095, B32B 27/08, B32B 27/30, B32B 27/36, B32B 27/40, A61K 9/70

(54) **Packaging material and package structure using the same**
Verpackungsmaterial und Verpackungsstruktur damit
Matériau d'emballage et structure d'emballage l'utilisant

(30) Priority: 21.09.2012 JP 2012208811
(43) Date of publication of application: 26.03.2014
(73) Proprietor: Toyo Seikan Group Holdings, Ltd., Shinagawa-ku, Tokyo 1418627 (JP); NITTO DENKO CORPORATION, Ibaraki-shi, Osaka 567-8680 (JP)
(72) Inventor: Ohta, Yoshihiro, Yokohama-shi, Kanagawa 2400062 (JP); Ishizaki, Yoichi, Yokohama-shi, Kanagawa 2400062 (JP); Hanatani, Akinori, Ibaraki-shi Osaka 5678680 (JP); Sakamoto, Sachiko, Ibaraki-shi, Osaka 5678680 (JP); Mukobata, Tsuyoshi, Ibaraki-shi, Osaka 5678680 (JP); Iwao, Yoshihiro, Ibaraki-shi, Osaka 5678680 (JP)
(74) Representative: Chapman, Paul Gilmour

(56) References cited:
- WO-A1-2010/029977
- WO-A1-2012/023555
- WO-A2-02/38134
- JP-A- 2006 305 975
- US-A1- 2007 128 262

## Description

### [Technical Field]

The present invention relates to a packaging material for a patch, and more particularly to a packaging material for a patch which contains an easily oxidizable component and an oily component in an adhesive and is susceptible to oxygen.

### [Background Art]

Patches, particularly transdermal patches having a drug in an adhesive layer are hermetically packaged with a sealed packaging material with the view to keeping the quality during storage or other purposes. However, when the patch having an easily oxidizable component and an oily component in an adhesive is packaged with a packaging material, the easily oxidizable component and the oily component are adsorbed or transferred to the packaging material due to contact of the patch with the inner surface of the packaging material, which leads to change in the contents of the easily oxidizable component and the oily component in the adhesive change, and accordingly change in the adhesiveness. In addition, it has been pointed out that the transdermal patch has problems such as decreased transdermal absorption of a contained drug and decreased pharmacological effect due to the adsorption of the contained drug itself.

In order to address the above problems, it has been proposed that the inner surface of a packaging material is made of ethylene/vinyl alcohol copolymer or acrylonitrile/methyl acrylate copolymer (Japanese Patent Application Publication JP-A-5-305108). As a patch excellent in stability of a medicinal component susceptible to oxygen or the like and an excellent method for stably storing the medicinal component of the patch, there have been proposed a patch enclosed in a packaging container having a deoxidizing ability and a patch storage method including enclosing the patch in a packaging container having a deoxidizing ability (Japanese Patent Application Publication (JP-A-11-47233). US2007128262 discloses a packaging material containing a patch comprising an inner layer made of polyacrylonitrile having heat sealing properties and an oxygen barrier layer made of aluminum foil as an outer layer. The polyacrylonitrile inner layer inhibits the migration of the drug from the patch onto the surface of the inner layer.

It has been, however, difficult to realize a packaging material that prevents an easily oxidizable component and an oily component in a patch from being transferred to a packaging material, and satisfies the stability of a medicinal component susceptible to oxygen in the patch at the same time.

### [Summary of Invention]

### [Technical Problems]

An object of the present invention is to provide a packaging material that is capable of long term storage by preventing an easily oxidizable component and an oily component in an adhesive of a patch from being adsorbed or transferred to the packaging material, and also preventing discoloration and decrease in drug effect due to oxidization.

### [Solution to Problems]

The present invention provides a packaging bag having a patch packaged with a packaging material, wherein the packaging material comprises:
an inner layer made of unstretched isophthalic acid modified polyethylene terephthalate having heat sealing properties;
an oxygen-absorbing layer containing an oxygen-absorbing resin having an unsaturated alicyclic structure as an oxygen-absorbing part; and
an oxygen barrier layer made of aluminum foil as an outer layer, and
the patch includes an adhesive layer containing an oily component.
Also disclosed herein is a packaging material for a patch including: an inner layer made of polyethylene terephthalate having heat sealing properties; an oxygen-absorbing layer that contains an oxygen-absorbing resin having an unsaturated alicyclic structure as an oxygen-absorbing part; and an oxygen barrier layer made of aluminum foil as an outer layer.

In the packaging material of the present invention, it is preferable that:
(1) the oxygen-absorbing layer is an oxygen-absorbing adhesive layer that includes an oxygen-absorbing polyester polyol having an unsaturated alicyclic structure as an oxygen-absorbing part and an isocyanate curing agent;
(2) the oxygen-absorbing polyester polyol is a polyester polyol having a structural unit derived from a tetrahydrophthalic acid or a derivative thereof; and
(3) the patch is a transdermal preparation that has an adhesive layer containing a transdermal drug formed on one side of a support.

Furthermore, in a package structure of the present invention, it is preferable that:
(4) a patch is packaged with the above packaging material; and
(5) the patch is a transdermal preparation that has an adhesive layer containing a transdermal drug formed on one side of a support.

### [Effects of the Invention]

The present invention realizes the packaging material that is capable of long term storage by preventing the easily oxidizable component and the oily component in the adhesive of the patch from being adsorbed or transferred to the packaging material, and also preventing discoloration and decrease in drug effect due to oxidization.

### [Brief Description of the Drawings]

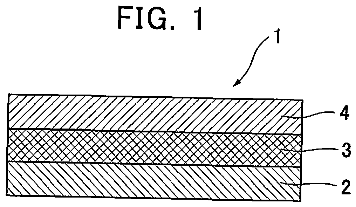

Fig. 1 is a cross-sectional view of the packaging material of a packaging bag of the present invention.

### [Description of Embodiments]

The packaging material for a patch of the present invention includes an inner layer made of polyethylene terephthalate having heat sealing properties, an oxygen-absorbing layer that contains an oxygen-absorbing resin having an unsaturated alicyclic structure as an oxygen-absorbing part, and an oxygen barrier layer made of aluminum foil as an outer layer.

As the polyethylene terephthalate resin having heat sealing properties, a copolymerized polyethylene terephthalate resin is employed. Since having poor absorption capacity for an oily component and moderate gas permeability, the polyethylene terephthalate resin does not inhibit a deoxidizing function of the oxygen-absorbing intermediate layer in the container. Accordingly, the polyethylene terephthalate resin is excellent as the inner layer of the packaging material that prevents the oily component in the patch from being transferred to the packaging material, and achieves the stability of a medicinal component susceptible to oxygen in the patch at the same time. The polyethylene terephthalate resin is a copolymerized polyethylene terephthalate resin that is modified with isophthalic acid. Also disclosed is a copolymerized polyethylene terephthalate resin that is modified with adipic acid. This resin is excellent in heat sealing properties due to its low crystallinity.

The inner layer made of polyethylene terephthalate is not stretched. The polyethylene terephthalate stretched at a high rate is not preferred since it loses the heat sealing properties due to oriented crystallization.

The oxygen-absorbing layer includes an oxygen-absorbing resin having an unsaturated alicyclic structure as an oxygen-absorbing part. The oxygen-absorbing resin having an unsaturated alicyclic structure as an oxygen-absorbing part is an excellent packaging material for a patch since it generates less cracked gas associated with autoxidation reactions, i.e., oxygen absorption reactions, than oxygen absorbents that include unsaturated fatty acid or chain hydrocarbon polymer having unsaturated groups and generates a larger amount of cracked gas. Suppressed generation of the cracked gas such as aldehyde, ketone, carboxylic acid, and alcohol reduces deterioration in quality of the patch through chemical reactions between the cracked gas and the components of the patch, which enables long term storage of the patch.

The oxygen-absorbing resin having an unsaturated alicyclic structure as an oxygen-absorbing part is any of polymers having a cyclohexene ring, for example, a condensation polymer using an ethylene-methyl acrylate-cyclohexyl methyl acrylate copolymer or tetrahydrophthalic anhydride derivative as a material. Cyclization reaction products of conjugated diene polymer can be also used.

Preferred oxygen-absorbing resins having an unsaturated alicyclic structure as an oxygen-absorbing part include resins obtained by polymerization of a material including a monomer selected from the group consisting of the following monomers (i) and (ii):
(i) a monomer that has an unsaturated alicyclic structure including a carbon atom bonded to both of the groups having the following structures (a) and (b) and further bonded to one or two hydrogen atoms in the unsaturated alicyclic structure;
   (a) a carbon-carbon double bond group in the unsaturated alicyclic structure,
   (b) a group selected form the group consisting of functional groups including a heteroatom, linking groups derived from the functional groups, a carbon-carbon double bond group, and aromatic rings; and
(ii) a monomer in which a carbon atom adjacent to a carbon-carbon double bond in the unsaturated alicyclic structure is bonded to an electron-donating substituent and a hydrogen atom, another carbon atom adjacent to the carbon atom is bonded to a functional group including a heteroatom or a linking group derived from the functional group, and the electron-donating substituent and the functional group including a hetero-atom or the linking group derived from the functional group are located in cis-position.

The oxygen-absorbing resin obtained by polymerization of the material including the monomers (i) and (ii) is preferably polyester having a structural unit derived from a tetrahydrophthalic acid or a derivative thereof such as a tetrahydrophthalic anhydride.

An acid component having the structure (i) is a Δ²-tetrahydrophthalic acid or a derivative thereof, a Δ³-tetrahydrophthalic acid or a derivative thereof, a A²⁻tetrahydrophthalic anhydride or a derivative thereof, or a Δ³-tetrahydrophthalic anhydride or a derivative thereof. A preferred one is a Δ³-tetrahydrophthalic acid or a derivative thereof, or a Δ³-tetrahydrophthalic anhydride or a derivative thereof. A particularly preferred one is a 4-methyl-Δ³-tetrahydrophthalic acid or a derivative thereof, or a 4-methyl-Δ³-tetrahydrophthalic anhydride or a derivative thereof. The 4-methyl-Δ³-tetrahydrophthalic anhydride can be obtained by, for example, conformational isomerization of an isomeric mixture including a 4-methyl-Δ⁴-tetrahydrophthalic anhydride, which is produced by the reaction of maleic anhydride and a C5 fraction of naphtha mainly including isoprene, and is industrially manufactured.

A particularly preferable acid component having the structure (ii) is a cis-3-methyl-Δ⁴-tetrahydrophthalic acid or a derivative thereof, or a cis-3-methyl-Δ⁴-tetrahydrophthalic anhydride or a derivative thereof. The cis-3-methyl-Δ⁴-tetrahydrophthalic anhydride can be obtained by, for example, the reaction of maleic anhydride with a C5 fraction of naphtha mainly including trans-piperylene, and is industrially manufactured.

The oxygen-absorbing layer is preferably a layer with an oxygen-absorbing adhesive. The oxygen-absorbing adhesive layer preferably contains a main agent of an oxygen-absorbing polyester polyol having an unsaturated alicyclic structure as an oxygen-absorbing part, and an isocyanate curing agent.

Various kinds of well-known aromatic, aliphatic, or alicyclic isocyanates can be used as isocyanate curing agents. Examples of aromatic diisocyanates include a 4,4'-diphenylmethane diisocyanate and a tolylene diisocyanate. Examples of aliphatic diisocyanates include a hexamethylene diisocyanate, a xylylene diisocyanate, and a lysine diisocyanate. Examples of alicyclic diisocyanates include a cyclohexane-1,4-diisocyanate, a isophorone diisocyanate, dicyclohexyl methane-4,4'-diisocyanate, and a dimer diisocyanate which is obtained by converting a carboxyl group of dimer acid into an isocyanate group. Furthermore, these isocyanates can be also used as a trimethylolpropane adduct, isocyanurate, biuret, or other forms. The above isocyanates and derivatives of the isocyanates may be used alone, or may be used in combination of two or more.

The content of the isocyanate curing agent is preferably 3 to 30 phr, more preferably 5 to 20 phr, and still more preferably 7 to 15 phr, based on the weight of the solid content, with respect to the oxygen-absorbing resin of the main agent. A too small amount of the isocyanate curing agent added results in insufficient adhesiveness and cohesion, and a too large amount thereof results in smaller pars of the oxygen-absorbing component per unit weight of the resin and thus insufficient oxygen-absorbing capability. When the flexibility of the resin is significantly decreased by curing, the oxygen absorption reaction does not easily proceed and the oxygen-absorbing capability decreases.

The oxygen-absorbing polyester polyol having an unsaturated alicyclic structure as an oxygen-absorbing part, which is used for the oxygen-absorbing adhesive layer, is preferably a polyester polyol having a structural unit derived from a tetrahydrophthalic acid or a derivative thereof.

As the oxygen barrier layer, aluminum foil is used due to its excellent barrier properties not only against oxygen but also against water and light.

For manufacture of the packaging material, extrusion molding known per se can be used. For example, a stacked body can be formed by extrusion molding with multiple multilayer dies using a certain number of extruders according to the resin types . The stacked body can be also manufactured by extrusion coating or sandwich lamination, or dry lamination of preformed films.

The stacked body can be made into packaging bags such as general three-side or four-side sealed pouches, gusseted pouches, standing pouches, and pillow pouches. The bag-making can be carried out by well-known bag-makings.

The patch packaged with the above packaging material includes a support layer and an adhesive layer, in which a drug is included in the adhesive layer to treat or prevent various diseases through transdermal absorption of the drug. The drug here is not particularly limited, and both systemically acting drugs and locally acting drugs can be used. Examples of the drug include adrenocorticosteroids, non-steroid anti-inflammatory agents, antirheumatics, hypnotics, antipsychotics, antidepressants, mood stabilizers, psychostimulants, anxiolytics, antiepileptics, migraine medications, Parkinson's disease drugs, muscarinic receptor antagonists, restless legs syndrome drugs, cerebral circulation metabolism-improving agents, anti-dementia drugs, autonomic drugs, muscle relaxants, antihypertensives, diuretics, hypoglycemics, hyperlipemic drugs, gout drugs, general anesthetics, local anesthetics, antibiotics, antifungals, antivirotics, antiparasitics, vitamins, antianginals, vasodilators, antiarrhythmics, antihistamines, mediator release inhibitors, leukotriene antagonists, sexual hormones, thyroid hormones, antithyroids, antitumor agents, antiemetics, anti-vertigenous drugs, bronchodilators, antitussives, expectorants, stop smoking aids, and antiosteoporotic agents . These drugs may be used in a free form or may be used in a salt form. These drugs may be used alone or in a mixture of two or more.

The patch used for the present invention includes an easily oxidizable component susceptible to oxygen. Specifically, the easily oxidizable component is a compound containing at least one of amino group, benzyl group, thiol group, and carbonyl group in the molecule. For example, compounds having an amine, amide, cyclic amine, lactone, or carboxylic acid structure are included therein.

The support layer is not particularly limited unless it is significantly uncomfortable to apply. Specifically, the support layer may be any of single films made of synthetic resins such as polyesters, polyolefins including polyethylene, polypropylene or the like, polyvinyl chloride, plasticized polyvinyl chloride, plasticized vinyl acetate-vinyl chloride copolymer, polyvinylidene chloride, ethylene-vinylacetate copolymer, cellulose acetate, ethyl cellulose, ethylene-ethyl acrylate copolymer, polytetrafluoroethylene, polyurethane, and ionomer resins, laminate films thereof, porous films and sheets, nonwoven fabrics, and woven fabrics which are made of rubber, the above synthetic resins, polyesters such as polyethylene terephthalate, polyamides such as nylon, laminates of these and the above synthetic resin film, and the like.

The patch is a transdermal preparation that preferably has the adhesive layer containing a transdermal drug formed one side of the support. It may also have a release-treated release film which is stacked on the opposite side to the adhesive layer.

The patch is in a planar flat form, and the planar shape is any of, but is not limited to, substantial rectangle as well as polygons such as triangle and pentagon, i.e., shapes outlined with substantial straight lines, shapes outlined with a curve such as ellipse and circle, combination of these and the like.

The size of the patch can be appropriately selected according to the applications and applied parts of the patch and the like. For example, when the patch is substantially rectangle-shaped, the length of its one side is 15 to 90 mm and the length of the other side is also 15 to 90 mm.

The patch usually has a total thickness of 50 to 2000 µm and preferably has a total thickness of 100 to 1000 µm. While the patch includes the support layer and the adhesive layer, the support layer usually has a thickness of 1 to 1000 µm and the adhesive layer usually has a thickness of 10 to 200 µm, and preferably has a thickness of 15 to 150 µm.

The adhesive layer contains an oily component. The contained oily component plasticizes the adhesive layer to impart a soft feeling, reducing skin irritation, and controls the transdermal absorption of the drug. The oily component is preferably in a liquid form at room temperature (25°C), and when a mixture of two or more of the oily components is used, it is preferably in a liquid form at room temperature (25°C) at the end. Examples of the oily components include higher alcohols such as oleyl alcohol and octyldodecanol; polyhydric alcohols such as glycerol, ethylene glycol, and polypropylene glycol; higher fatty acids such as caprylic acid and oleic acid; fatty acid esters such as isopropyl myristate, isopropyl palmitate, and ethyl oleate; polybasic acid ester such as diethyl sebacate and diisopropyl adipate; fatty acid esters of polyhydric alcohols, such as triisostearate diglyceryl, sorbitan monooleate , propylene glycol dicaprylate, and polyethylene glycol monolaurate and polyoxyethylene sorbitol tetraoleate; polyoxyethylene alkyl ethers such as polyoxyethylene lauryl ether; hydrocarbons such as squalane and liquid paraffin; vegetable oils such as olive oil and castor oil; silicone oils; pyrrolidones such as N-methyl pyrrolidone and N-dodecylpyrrolidone; and sulfoxides such as decyl methyl sulfoxide. These oily components may be used alone or in a mixture of two or more.

The shape of the packaging body for the patch of the present invention is not particularly limited as long as the packaging body can package the patch. Examples of the shape include substantial rectangles such as square and rectangle, as well as polygons such as triangle and pentagon, circle, ellipse and other figures. The shape of the packaging body for the patch and the shape of the patch packaged may be the same or different as long as individual package (sealing) of the patch is achieved.

### [Examples]

### 1. [Preparation of Polyester Polyol Including Unsaturated Alicyclic Structure]

Into a 3L separable flask equipped with a stirring device, a nitrogen introducing tube, and a Dean-Stark water separator, 898 g of an isomeric mixture of methyl tetrahydrophthalic anhydride (produced by Hitachi Chemical Co., Ltd.; HN-2200) containing 45 mol% of 4-methyl-Δ³-tetrahydrophthalic anhydride and 21 mol% of cis-3-methyl-Δ⁴-tetrahydrophthalic anhydride, 60 g of succinic anhydride (produced by Wako Pure Chemical Industries, Ltd.), 702 g of 1,4-butanediol (produced by Wako Pure Chemical Industries, Ltd.), 300 ppm of isopropyl titanate (produced by Kishida Chemical Co., Ltd.) as a polymerization catalyst, and 20 ml of toluene were charged and reacted for about 5 hours while removing water generated at 150°C to 200°C in a nitrogen atmosphere. Subsequently, toluene was removed from the reaction system and polymerization was then performed at 200°C to 220°C under a reduced pressure of 0.7 kPa for about 6 hours to obtain a polyester polyol. The obtained polyester polyol had a Mn of about 3400 and a Mw of 27100.

### 2. [Preparation of Solution of Oxygen-absorbing Adhesive]

The obtained polyester polyol was dissolved at 20 wt% in ethyl acetate, and an isocyanate curing agent (produced by Mitsui Chemicals; A-50) was added at 10 phr based on the solid content and shaken to prepare a solution of an oxygen-absorbing adhesive.

### 3. [Production of Packaging Material for Adhesive Patches]

The prepared adhesive solution was applied to the aluminum foil surface side of a dry laminate film substrate including a 12 µm biaxially-stretched PET film and a 7 µm aluminum foil by a #18 bar coater. A solvent in the adhesive was immediately evaporated in an electric oven of 100°C for 1 minute. Subsequently, the dry laminate film substrate and a 20 µm unstretched isophthalic acid modified PET film were attached to each other by pressure with a heating roll of 70°C such that the adhesive applied surface of the dry laminate film substrate faced the unstretched isophthalic acid modified PET film, and cured for 5 days under a nitrogen atmosphere of 35°C to obtain an oxygen-absorbing stacked film of biaxially-stretched PET film / aluminum foil / oxygen-absorbing adhesive (thickness: 4 µm) / unstretched PET film.

### 4. [Evaluation of Oxygen-absorbing Capability]

A specimen of 2 cm × 10 cm which was cut out from an oxygen-absorbing stacked film specimen was charged together with a humidity controlling liquid containing a glycerin solution into an oxygen-impermeable steel foil stack cup having an inner volume of 85 cm³, tightly sealed with a lid of aluminum foil stacked film by heat sealing, and stored under an atmosphere of 22°C. The humidity in the cup was controlled to 50% RH with the humidity controlling liquid. The oxygen concentration in the cup after the storage in 7 days and 30 days was determined with a micro gas chromatograph (produced by Agilent Technologies, Inc. ; M200) to obtain the amount of oxygen absorbed per m² of the film. As a result, the amount of oxygen absorbed was 170 ml/m² in 7 days and 450 ml/m² in 30 days.

### 5. [Production of Adhesive patch]

2-ethylhexyl acrylate, N-vinylpyrrolidone, and acrylic acid were copolymerized in the ratio of 74: 22: 4 (by weight) to prepare a polymer having an absolute molecular weight of 1.8 × 10⁶. Then, 41.5 parts by weight (solid content) of this polymer, 50.9 parts by weight of isopropyl myristate (IPM), 7.4 parts by weight of propranolol, and 0.2 parts by weight of ethylacetoacetate aluminum diisopropylate were mixed well while controlling the viscosity with ethyl acetate to obtain an adhesive solution.

This adhesive solution was applied to the siliconized biaxially-stretched PET film so that the thickness after drying was 60 µm. After drying, the biaxially-stretched PET film adhered thereto as a support. This was heated at 70°C under a nitrogen atmosphere for 48 hours and then cut into a size of 10 cm² to obtain a patch.

### [Example 1]

The obtained oxygen-absorbing stacked film was cut into two squares of 7 cm × 7 cm, which were then layered such that their unstretched PET film layers faced each other. One patch produced was placed therebetween and four sides of the film were heat-sealed with a seal width of 5 mm to produce a package structure containing the patch and having an inner volume of 2 ml.

The change in quality of the patch was evaluated according to the following method. The results are shown in Table 1.

### [Comparative Example 1]

A package structure containing a patch was produced and evaluated as in Example 1 except that a dry laminate stacked film of biaxially-stretched PET film / aluminum foil / unstretched PET film, which did not have oxygen-absorbing capability, was used as a packaging material instead of the oxygen-absorbing stacked film. The results are shown in Table 1.

### [Comparative Example 2]

A package structure containing a patch was produced and evaluated as in Example 1 except that an oxygen-absorbing stacked film of biaxially-stretched PET film / aluminum foil / oxygen-absorbing adhesive (thickness: 4 µm) / LDPE film, which was produced by laminating a 30 µm LDPE film instead of a 20 µm unstretched isophthalic acid modified PET film, was used as a packaging material. The results are shown in Table 1.

### [Test Example]

After storing the package structures of Example 1, Comparative Example 1, and Comparative Example 2 at 50°C for 3 months, the content of the drug (propranolol), the impurity generation rate, the color, and the content of IPM were determined for each patch and compared with the values before the storage (initial values).

The content of the drug was obtained by analyzing a methanol extract of the patch by liquid chromatography (HPLC) and evaluated based on the ratio of the content after the storage to that before the storage.

The impurity generation rate was evaluated based on the difference between the ratio after the storage and the initial ratio (increase after the storage) of the total peak area of other components to the drug peak area in the HPLC chromatogram obtained for determining the content of the drug.

The color was determined with a color difference meter and evaluated based on the color difference ΔE* between the initial color and the color after the storage in the L*a*b* color system.

The content of IPM was determined by analyzing a hexane extract of the patch by gas chromatography, and evaluated based on the ratio of the content after the storage to the initial content.

**Table 1**

| | Content of Drug | Impurity Generation Rate | Color | Content of IPM |
|---|---|---|---|---|
| Example 1 | 95% | 1.5% | 0.0 | 98% |
| Comparative Example 1 | 87% | 4.1% | 5.0 | 98% |
| Comparative Example 2 | 80% | 1.9% | 0.3 | 58% |

As shown in Table 1, Example 1 achieves favorable results for all evaluation items. On the other hand, Comparative Example 1 is found to have significant decomposition of the drug, because the decrease in content of the drug, the increase in impurity generation rate, and the color change are relatively large. In Comparative Example 2, the impurity generation rate and the color change are relatively small, which indicates that the effect of the oxygen-absorbing adhesive was exerted, but the decreases in contents of IPM and the drug are large, which indicates that they were adsorbed to the inner layer of the packaging material.

### [Description of Reference Numerals]

- 1: Packaging Material
- 2: Inner Layer
- 3: Oxygen-absorbing Intermediate Layer
- 4: Oxygen Barrier Layer

## Claims

1. A packaging bag having a patch packaged with a packaging material, wherein the packaging material comprises:
an inner layer made of unstretched isophthalic acid modified polyethylene terephthalate having heat sealing properties;
an oxygen-absorbing layer containing an oxygen-absorbing resin having an unsaturated alicyclic structure as an oxygen-absorbing part; and
an oxygen barrier layer made of aluminum foil as an outer layer, and the patch includes an adhesive layer containing an oily component.

2. The packaging bag according to claim 1, wherein the patch is a transdermal preparation in which an adhesive layer containing a transdermal drug is formed on one side of a support.

3. The packaging bag according to claim 1, wherein
the oxygen-absorbing layer is an oxygen-absorbing adhesive layer that contains an oxygen-absorbing polyester polyol having an unsaturated alicyclic structure as an oxygen-absorbing part, and an isocyanate curing agent.

4. The packaging bag according to claim 3, wherein
the oxygen-absorbing polyester polyol is a polyester polyol having a structural unit derived from a tetrahydrophthalic acid or a derivative thereof.

5. The packaging bag according to any one of claims 1-4, wherein the oily component is selected from the group consisting of higher alcohols, polyhydric alcohols, higher fatty acids, fatty acid esters, polybasic acid ester, fatty acid esters of polyhydric alcohols, polyoxyethylene alkyl ethers, hydrocarbons, vegetable oils, silicone oils, pyrrolidones, sulfoxides, and combinations thereof.

6. The packaging bag according to any one of claims 1-4, wherein the oily component comprises isopropyl myristate.

## Patentansprüche

1. Verpackungsbeutel mit einem mit einem Verpackungsmaterial verpackten Pflaster, wobei das Verpackungsmaterial umfasst:
eine innere Schicht aus ungedehntem Isophthalsäure-modifiziertem Polyethylenterephthalat mit Heißsiegeleigenschaften;
eine sauerstoffabsorbierende Schicht, die ein sauerstoffabsorbierendes Harz mit einer ungesättigten alicyclischen Struktur als sauerstoffabsorbierenden Teil enthält; und
eine Sauerstoffbarriereschicht aus Aluminiumfolie als äußere Schicht, und wobei das Pflaster eine Klebstoffschicht einschließt, die eine ölige Komponente enthält.

2. Verpackungsbeutel nach Anspruch 1, wobei das Pflaster ein transdermales Präparat ist, in dem eine Haftschicht, die ein transdermales Arzneimittel enthält, auf einer Seite eines Trägers ausgebildet ist.

3. Verpackungsbeutel nach Anspruch 1, wobei
die sauerstoffabsorbierende Schicht eine sauerstoffabsorbierende Haftschicht ist, die ein sauerstoffabsorbierendes Polyesterpolyol mit einer ungesättigten alicyclischen Struktur als sauerstoffabsorbierenden Teil und ein Isocyanat-Härtungsmittel enthält.

4. Verpackungsbeutel nach Anspruch 3, wobei
das sauerstoffabsorbierende Polyesterpolyol ein Polyesterpolyol mit einer Struktureinheit ist, die von einer Tetrahydrophthalsäure oder einem Derivat davon abgeleitet ist.

5. Verpackungsbeutel nach einem der Ansprüche 1 bis 4, wobei die ölige Komponente ausgewählt ist aus der Gruppe bestehend aus höheren Alkoholen, mehrwertigen Alkoholen, höheren Fettsäuren, Fettsäureestern, mehrbasischen Säureestern, Fettsäureestern von mehrwertigen Alkoholen, Polyoxyethylenalkylethern, Kohlenwasserstoffen, Pflanzenölen, Silikonölen, Pyrrolidonen, Sulfoxiden und Kombinationen davon.

6. Verpackungsbeutel nach einem der Ansprüche 1 bis 4, wobei die ölige Komponente Isopropylmyristat umfasst.

## Revendications

1. Sachet d'emballage pourvu d'un patch emballé avec un matériau d'emballage, dans lequel le matériau d'emballage comprend :
une couche intérieure faite de polytéréphtalate d'éthylène modifié à l'acide isophtalique non étiré ayant des propriétés de scellement thermique ;
une couche absorbant l'oxygène contenant une résine absorbant l'oxygène ayant une structure alicyclique insaturée en tant que partie absorbant l'oxygène ; et
une couche de barrière à l'oxygène faite de feuille d'aluminium en tant que couche extérieure, et le patch incluant une couche adhésive contenant un composant huileux.

2. Sachet d'emballage selon la revendication 1, dans lequel le patch est une préparation transdermique, dans lequel une couche adhésive contenant un médicament transdermique est formée sur un côté du support.

3. Sachet d'emballage selon la revendication 1, dans lequel
la couche absorbant l'oxygène est une couche adhésive absorbant l'oxygène qui contient un polyol polyester absorbant l'oxygène ayant une structure alicyclique insaturée en tant que partie absorbant l'oxygène, et un agent de durcissement isocyanate.

4. Sachet d'emballage selon la revendication 3, dans lequel
le polyol polyester absorbant l'oxygène est un polyol polyester ayant une unité structurelle dérivée à partir d'un acide tétrahydrophtalique ou d'un dérivé de celui-ci.

5. Sachet d'emballage selon l'une quelconque des revendications 1 à 4, dans lequel le composant huileux est sélectionné à partir du groupe constitué d'alcools supérieurs, d'alcools polyhydriques, d'acides gras supérieurs, d'esters d'acides gras, d'esters d'acides polybasiques, d'esters d'acides gras d'alcools polyhydriques, d'éthers alkyliques de polyoxyéthilène, d'hydrocarbones, d'huiles végétales, d'huiles silicones, de pyrrolidones, de sulfoxides, et de combinaisons de ceux-ci.

6. Sachet d'emballage selon l'une quelconque des revendications 1 à 4, dans lequel le composant huileux comprend du myristate d'isopropyle.
